# EUROPÄISCHE PATENTSCHRIFT

(11) **EP 0 888 759 B1**
(45) Veröffentlichungstag und Bekanntmachung des Hinweises auf die Patenterteilung: **02.01.2003**
(21) Anmeldenummer: 97810445.3
(22) Anmeldetag: 04.07.1997
(51) Int. Cl.: A61F 2/46

(54) **Instrument zum Einbringen eines Einsatzes eines Implantats in die zugehörige Schale**
Instrument for introduction of an insert of an implant into the associated shell
Instrument pour l'introduction d'une pièce d'insertion d'un implant dans la coupe associée

(43) Veröffentlichungstag der Anmeldung: 07.01.1999
(73) Patentinhaber: Sulzer Orthopädie AG, 6341 Baar (CH)
(72) Erfinder: Peter, Rolf, 8050 Zürich (CH); Willi, Thomas, 8405 Winterthur (CH); Wymann, Burkhard, 8353 Elgg (CH)
(74) Vertreter: Manitz, Finsterwald & Partner Gbr

(56) Entgegenhaltungen:
- EP-A- 0 453 694
- WO-A-94/21199
- FR-A- 2 676 172
- GB-A- 2 299 758
- US-A- 5 098 437
- US-A- 5 540 697

## Beschreibung

Die Erfindung betrifft ein Instrument zum Einbringen eines Einsatzes eines Implantats, insbesondere einer künstlichen Hüftgelenkpfanne, in die zugehörige Schale dieses Implantats gemäss dem Oberbegriff des unabhängigen Patentanspruchs.

Einige Implantate, insbesondere aber künstliche Hüftgelenkpfannen, umfassen eine Schale, beispielsweise aus Titan, die am Beckenknochen befestigt wird, sowie einen Einsatz (ein "Inlay"), der in die Schale eingesetzt wird und der auf seiner von der Schale weg weisenden Fläche eine konkave Gleitfläche für die Schaftkugel aufweist. Der Einsatz selbst kann aus Kunststoff (z.B. Polyethylen), aus Metall, aus Keramik oder aus einer Kombination derartiger Werkstoffe mit anderen Werkstoffen hergestellt sein.

Nach dem Einbringen des Einsatzes in die Schale muss der Einsatz mit der Schale fest verbunden werden, soll aber bei Bedarf auch wieder aus der Schale lösbar sein (damit er ggf. ausgetauscht werden kann). Das Einbringen des Einsatzes in die Schale hinein erfolgt im Operationssaal. Beim Implantieren einer kompletten Hüftgelenkpfanne muss zunächst die Schale der Hüftgelenkpfanne am Beckenknochen des Patienten befestigt werden, anschliessend kann der Einsatz in die Schale eingebracht werden. Beim Austauschen des Einsatzes hingegen wird keine neue Schale am Beckenknochen befestigt, sondern es wird nur der alte Einsatz aus der Schale entfernt und anschliessend ein neuer Einsatz eingesetzt.

Die Verbindung von Einsatz und Schale kann auf unterschiedliche Weise ausgestaltet sein, ein Beispiel dafür ist eine Schnappverbindung zwischen Einsatz und Schale, mit anderen Worten gesagt, schnappt der Einsatz beim Einbringen in die Schale hinein in der Schale ein und ist somit mit ihr fest verbunden, aber bei Bedarf auch wieder lösbar. Andere Verbindungen von Einsatz und Schale beruhen darauf, dass sowohl die Schale auf ihrer Innenfläche als auch der Einsatz auf seiner Aussenfläche konisch zulaufend ausgebildet sind, die feste Verbindung von Einsatz und Schale somit über den Konus (also über Reibungskräfte) erfolgt. Auch Kombinationen dieser beiden Verbindungsarten sind bekannt. Das Einbringen und Befestigen des Einsatzes erfolgt im Operationssaal unter sterilen Bedingungen.

Die WO-A-94 21199 betrifft ein Einsatzstück mit einem Auslösemechanismus zum Positionieren einer Hüftgelenkspfannenprothese. Die Vorrichtung umfasst einen Positionierstab mit einem an diesem zu befestigenden Einsatz, der federnde Lamellen aufweist, deren freie Enden in einer im Hohlraum der zu implantierenden halbkugelförmigen Pfanne ausgebildeten Nut in Eingriff gebracht werden können. Auf dem Positionierstab befindet sich ein Schiebstück, das, wenn es in axialer Richtung über die Lamellen geschoben wird, diese in radialer Richtung nach innen verschiebt, so dass die Enden aus der Nut austreten können, wodurch die Pfanne von dem Einsatzstück freigegeben wird.

Die US 5 540 697 offenbart eine Einbauvorrichtung für eine Pfannenprothese mit einem Werkzeugkörper, einem hohlen Außenkörper, in dem der Werkzeugkörper verschiebbar ist, und am Werkzeugkörper an einem Ende angebrachten Eingriffsarmen, die zwischen einer ausgefahrenen Stellung und einer zurückgezogenen Stellung verschiebbar sind, wobei sie in der ausgefahrenen Stellung am Umfang des Innenraums eines Pfannenimplantats in Eingriff stehen. Die Eingriffsarme können über einen am Außenkörper vorgesehenen Hebelmechanismus von der Pfanne gelöst werden, so dass die Einbauvorrichtung und die Pfanne trennbar sind.

Es ist eine Aufgabe der Erfindung, ein Instrument vorzuschlagen, mit welchem Einsätze von Implantaten, insbesondere von künstlichen Hüftgelenkpfannen, auf einfache und zuverlässige Weise in eine entsprechende Schale eingesetzt und dort befestigt werden können. Gleichzeitig soll das Einsetzen einigermassen schnell und unabhängig von der jeweiligen Lage des Patienten auf dem Operationstisch möglich sein. Der Einsatz soll mit Hilfe des Instruments so platziert werden, dass ein falsches Einsetzen praktisch unmöglich ist.

Erfindungsgemäss wird die Aufgabe mit einem Instrument gelöst, wie es durch die Merkmale des unabhänigen Patentanspruchs charakterisiert ist. Erfindungsgemäß wird der Einsatz von dem Instrument sicher zwischen den Lamellen gehalten und kann in jeder beliebigen Lage des Patienten problemlos zur Schale geführt werden. Mit Hilfe des Betätigungsorgans kann der Einsatz in die Schale hinein freigegeben und dort befestigt werden, sodass das Einsezten insgesamt einfach, schnell und zuverlässig im Operationssaal erfolgen kann und ein falsches Einsetzen praktisch nicht möglich ist.

Dabei weist der an der Innenwand der Lamellen vorgesehene Konus an der Stelle seines grössten Durchmessers einen Durchmesser auf, der kleiner oder gleich dem grössten Aussendurchmesser des Konus auf der Aussenwand des Einsatzes ist. Die Lamellen werden beim Einführen des Einsatzes zwischen die Lamellen gespreizt und der auf den Innenflächen der Lamellen vorgesehene Konus bildet mit dem Konus auf der Aussenfläche des Einsatzes einen festen Sitz, sodass der Einsatz sicher zwischen den Lamellen gehalten wird. Ist der Durchmesser des Konus auf der Innenwand der Lamellen an der Stelle, wo er seinen grössten Durchmesser aufweist, gerade gleich gross wie der grösste Aussendurchmesser des Einsatzes, so passt der Einsatz genau zwischen die Lamellen und wird dort gehalten.

Bei einer Weiterbildung ist der Konuswinkel der Konusfläche grösser oder gleich dem Konuswinkel des Konus auf der Aussenwand des Einsatzes. Idealerweise sind die beiden Konuswinkel gleich gross, sodass der Einsatz praktisch über seine gesamte konische Fläche von den Lamellen festgehalten wird.

In einem weiteren Ausführungsbeispiel ist am proximalen Ende der die Konusfläche definierenden Flächen auf der Innenwand der Lamellen, also dort, wo der an der Innenwand der Lamellen vorgesehen Konus seinen grössten Durchmesser aufweist, jeweils ein in radialer Richtung nach innen vorstehender Vorsprung vorgesehen. Dieser Vorsprung bildet einen Anschlag für den Einsatz, der Einsatz wird also zwischen den Lamellen gegen den Anschlag gedrückt, sodass er immer in einer genau definierten (und üblicherweise zentrierten) Position gehalten wird.

Ein weiteres Ausführungsbeispiel zeichnet sich dadurch aus, dass das distale Ende der die Konusfläche definierenden Fläche der jeweiligen Lamelle mit einer Fase versehen ist. Diese Fase erleichtert das Zuführen des Einsatzes zwischen die Lamellen, zwischen denen dann der Einsatz gehalten wird. Ausserdem resultiert aus dem Zuführen des Einsatzes über den von der Fase und der anschliessenden konischen Fläche gebildeten Vorsprung hinweg die Schnappverbindung zwischen den Lamellen und dem Einsatz.

Bei einem weiteren Ausführungsbeispiel ist der Stab im wesentlichen hohlzylindrisch ausgebildet. Das Betätigungsorgan zum Freigeben des Einsatzes weist eine Schubstange mit einem an ihrem distalen Ende befestigten Stössel auf, wobei die Schubstange in dem hohlzylindrischen Stab geführt und in Längsrichtung gegen die Kraft einer Rückstellfeder bewegbar ist. (Die Rückstellfeder ist nicht zwingend notwendig, erhöht aber die Benutzerfreundlichkeit). Bei Betätigung der Schubstange taucht der Stössel zwischen die Lamellen ein und stösst den zwischen den Lamellen festgehaltenen Einsatz aus. Anschliessend wird die Schubstange mitsamt dem Stössel mittels der Rückstellfeder wieder in ihre Ruhestellung bewegt. Dieses Ausführungsbeispiel ist konstruktiv einfach, einfach in der Handhabung und darüberhinaus auch einfach montierbar und demontierbar. Dies wiederum ist vorteilhaft im Hinblick darauf, dass die Instrumente regelmässig sterilisiert werden müssen

Um die Montierbarkeit und Demontierbarkeit des Instruments noch weiter zu vereinfachen bzw. auch das Sterilisieren zu vereinfachen, kann die Haltevorrichtung einen separaten Haltekopf umfassen, der mit dem Stab lösbar verbunden ist und an dem die Lamellen vorgesehen sind. Bei diesem Ausführungsbeispiel ist das Instrument praktisch vollständig in seine Einzelteile zerlegbar und dadurch auch auf einfache Weise sterilisierbar. Ausserdem wird auch die Fertigung der einzelnen Teile eines solchen Instruments vereinfacht. Der Haltekopf kann beispielsweise mittels eines Innengewindes auf einem entsprechenden Aussengewinde auf der Stange festgeschraubt werden.

Weitere vorteilhafte Aspekte der Erfindung ergeben sich aus der nachfolgenden Beschreibung mit Hilfe der Zeichnung. Es zeigen:
- Fig. 1: ein Ausführungsbeispiel des erfindungsgemässen Instruments in Ruhestellung und ohne einen Einsatz für die Schale einer Hüftgelenkpfanne,
- Fig. 2: das Ausführungsbeispiel gemäss Fig. 1, jedoch mit einem Einsatz für eine ebenfalls dargestellte Schale einer Hüftgelenkpfanne,
- Fig. 3: die Einzelheit III der Fig. 2,
- Fig. 4: das Ausführungsbeispiel gemäss Fig. 1 beim Einsetzen des Einsatzes in die Schale einer Hüftgelenkpfanne
und
- Fig. 5: das Instrument in der Stellung unmittelbar nach dem Einsetzen und Befestigen des Einsatzes in der Schale der Hüftgelenkpfanne.

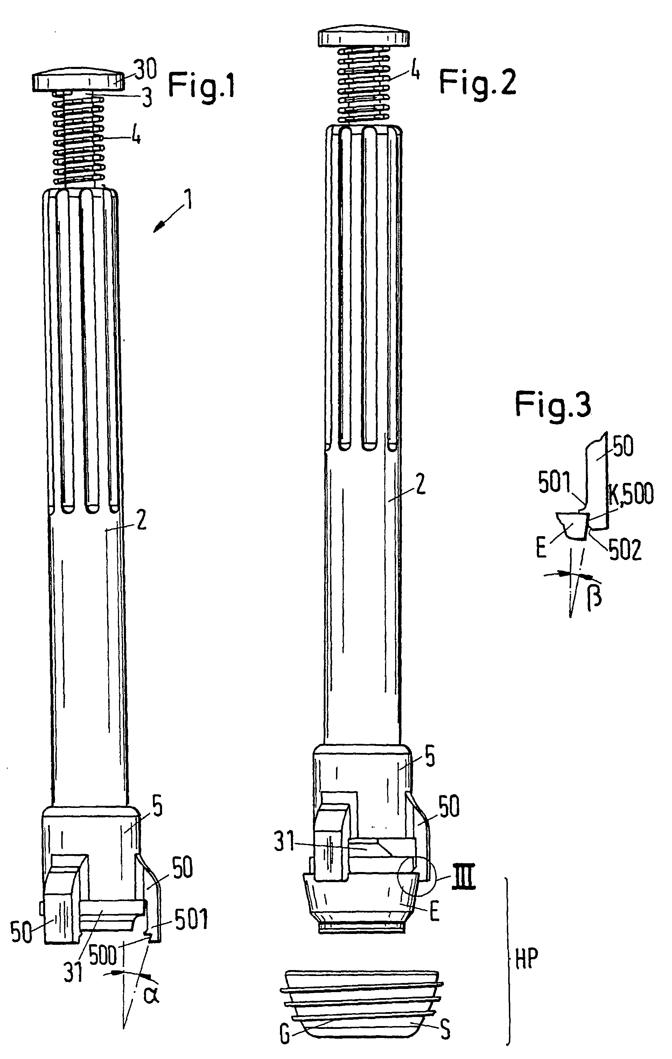

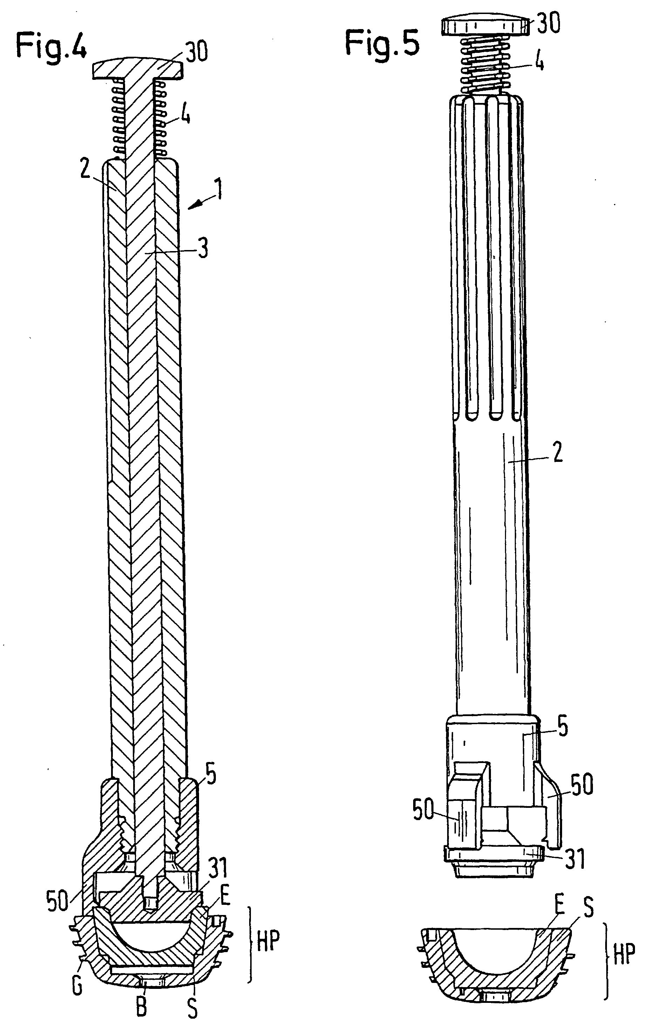

In dem in Fig. 1 dargestellten Ausführungsbeispiel des erfindungsgemässen Instruments 1 zum Einbringen eines Einsatzes E (Fig. 2) eines Implantats, insbesondere einer Hüftgelenkpfanne HP (Fig. 2), in die zugehörige Schale S (Fig. 2) des Implantats erkennt man einen länglichen hohlzylindrischen Stab 2, in welchem eine Schubstange 3 geführt ist. Die Schubstange 3 ist gegen die Kraft einer Rückstellfeder 4 in dem hohlzylindrischen Stab 2 bewegbar, indem die Bedienperson (z.B. der Chirurg bzw. Orthopäde) auf den proximal angeordneten Kopf 30 drückt. An seinem distalen Ende weist die Schubstange 3 einen Stössel 31 auf, der z.B. über eine Schraubverbindung (Fig. 4) mit der Schubstange 3 verbunden (damit auch lösbar und einfach sterilisierbar) ist. Weiterhin ist am distalen Ende des Instruments 1 eine Haltevorrichtung in Form eines separaten Haltekopfs 5 vorgesehen, der mit dem Stab 2 z.B. über eine Schraubverbindung (Fig. 4) verbunden ist (was die Sterilisierung der einzelnen Teile des Instruments weiter erleichtert). Der Haltekopf 5 weist an seinem distalen Ende drei federnde Lamellen 50 auf (jeweils um 120° in Umfangsrichtung versetzt), deren freies Ende jweils vom Stab 2 in axialer Richtung wegweist. Die Lamellen 50 weisen auf ihrer Innenwand eine in Richtung auf ihr freies (also in Richtung auf das distale) Ende zulaufende konische Fläche 500 auf d.h. die Flächen 500 definieren gemeinsam eine sich entgegen der Einbringrichtung verjüngende Konusfläche. Ferner weisen die Lamellen 50 am proximalen Ende dieser konisch zulaufenden Fläche 500, also dort, wo der Konus seinen grössten Durchmesser aufweist, einen in radialer Richtung nach innen vorstehenden Vorsprung 501 auf.

In Fig. 2 erkennt man das Ausführungsbeispiel des erfindungsgemässen Instruments gemäss Fig. 1, wobei allerdings der Einsatz E der Hüftgelenkpfanne HP zwischen den Lamellen 50 gehalten wird. Dieser Einsatz E wird mit Hilfe des Instruments 1 in die Schale S eingesetzt. In der Praxis kann man sich das so vorstellen, dass die Schale S bereits im Beckenknochen des Patienten verankert ist, wobei der Patient eben in einer für den Chirurgen bzw. Orthopäden günstigen Position auf dem Operationstisch liegt. Für die Beschreibung des erfindungsgemässen Instruments und dessen Funktionsweise wird jedoch vereinfachend hier jeweils nur die Schale S dargestellt (und auf die Darstellung z.B. des Beckenknochens verzichtet).

Man erkennt in Fig. 2 noch, dass die Schubstange 3 schon um ein Stück aus ihrer Ruhelage bewegt dargestellt ist, da der Stössel 31 bereits zu einem Teil zwischen die Lamellen 50 eingetaucht ist (man erkennt dies auch an der geringfügig komprimierten Rückstellfeder 4), jedoch noch nicht so weit, dass er den zwischen den Lamellen 50 gehaltenen Einsatz E in die Schale S hinein ausstösst.

Fig. 3 zeigt die Einzelheit III aus Fig. 2 in vergrösserter Darstellung. In Fig. 3 erkennt man besonders gut die Art und Weise, wie der Einsatz E zwischen den Lamellen gehalten wird, wobei hier der Einfachheit halber nur ein Ausschnitt aus einer Lamelle 50 und nur ein Ausschnitt aus dem Einsatz E dargestellt sind. Man erkennt, dass der Einsatz E auf seiner Aussenwand eine konische Fläche K aufweist, welche zusammen mit der konischen Fläche 500 auf der Innenwand der Lamelle 50 einen festen Sitz bildet. Das distale Ende der konischen Fläche 500 ist mit einer Fase 502 versehen, sodass beim Zuführen des Einsatzes zwischen die Lamellen 50 der Einsatz auf einfache Weise geführt zwischen die Lamellen 50 eingebracht werden kann und beim Zuführen über den von der Fase 502 und der konischen Fläche 500 gebildeten Vorsprung hinweg eine Schnappverbindung gebildet wird. Die federnd ausgebildeten Lamellen 50 federn beim Zuführen des Einsatzes E zwischen die Lamellen zurück, da der Durchmesser des Konus auf der Innenwand der Lamellen 50 am distalen Ende in jedem Fall geringer ist als der Durchmesser des Konus am proximalen Ende auf der Aussenwand des Einsatzes E (dort ist er nämlich am grössten). Am proximalen Ende (also dort, wo der Durchmesser am grössten ist) ist der Durchmesser des Konus auf der Innenwand der Lamelle 50 ebenfalls kleiner oder aber gerade eben gleich gross wie der grösste Aussendurchmesser des Konus auf der Aussenwand des Einsatzes E. Der Konuswinkel α (Fig. 1) des Konus auf der Innenwand der Lamelle 50 ist (etwas) grösser oder genau gleich dem Konuswinkel β (Fig. 3) des Konus auf der Aussenwand des Einsatzes E.

In Fig. 4 ist das Instrument 1 in einem Längsschnitt (praktisch um 180° um die Längsachse verdreht) dargestellt, wobei in der Darstellung gemäss Fig. 4 gerade ein vom Instrument 1 zwischen den Lamellen 50 fest gehaltener Einsatz E in die Schale S einer Hüftgelenkpfanne HP eingesetzt und dort befestigt wird. Man erkennt, dass die Schale S auf ihrer Aussenwand Gewindestücke G aufweist (siehe auch Fig. 2), mit deren Hilfe es möglich ist, die Schale S im (nicht dargestellten) Beckenknochen zu befestigen. Zusätzlich weist die Schale S aber auch eine zentrale Bohrung B auf, durch welche ein geeignetes Verankerungsmittel - z.B. eine (nicht dargestellte) Schraube - hindurchgeführt wird, wobei diese Schraube in den Beckenknochen geschraubt wird und somit die Schale S verankert wird.

Zum Einbringen des Einsatzes E in die bereits verankerte Schale S wird nun das Instrument 1 mit den flachen vorderen Enden der drei Lamellen 50 auf den oberen Rand der Schale S aufgesetzt. Anschliessend drückt der Chirurg oder Orthopäde auf den Kopf 30 am proximalen Ende der Schubstange 3, wodurch die Rückstellfeder 4 komprimiert wird und der Stössel 31 den zwischen den Lamellen 50 gehaltenen Einsatz E in die Schale S hineindrückt. In dem hier gezeigten Ausführungsbeispiel wird der Chirurg oder Orthopäde so lange auf den Kopf 30 am proximalen Ende der Schubstange 3 drücken, bis mittels der Schubstange 3 und dem Stössel 31 der Einsatz E ganz in die Schale S hineingedrückt worden ist und - bei diesem Ausführungsbeispiel - eine von den beiden weiteren konischen Flächen am unteren Ende von Schale S und Einsatz E gebildete Schnappverbindung eingeschnappt ist. Selbstverständlich braucht aber für den festen Sitz des Einsatzes E in der Schale S keine solche Schnappverbindung am unteren Ende von Schale S und Einsatz E vorgesehen zu sein. Eine für solche Fälle typischerweise vorgesehene Konusverbindung ist für einen festen Sitz des Einsatzes E in der Schale S absolut ausreichend.

Fig. 5 zeigt schliesslich einen vollständig in die Schale S eingepassten Einsatz E, wobei das beim Einbringen auf den oberen Rand der Schale S aufgesetzte Instrument bereits von der Hüftgelenkpfanne HP wieder abgehoben worden ist, der Kopf 30 am proximalen Ende der Schubstange 3 jedoch noch in gedrückter Position dargestellt ist, man erkennt dies einerseits an der noch komprimierten Rückstellfeder 4, andererseits an dem noch vollständig zwischen die Lamellen 50 (praktisch bis ans freie Ende der Lamellen) eingetauchten Stössel 31 am distalen Ende der Schubstange 3.

Aufgrund des Einsatzes des Instruments im Operationssaal müssen die einzelnen Teile des Instruments sterilisierbar sein. Während die Schubstange 3 mit dem Kopf 30 aus einem nichtrostenden Metall, z.B. einem nichtrostenden Stahl, hergestellt sein können, kann der hohlzylindrische Stab 2 beispielsweise aus Polyoxymethylen (POM-C) hergestellt sein. Der Haltekopf 5 mit den drei flexiblen Lamellen (es können natürlich auch mehr als drei Lamellen vorgesehen sein) kann beispielsweise aus Polyetherimid hergestellt sein. Schliesslich kann der Stössel 31 ebenfalls aus einem sterilisierbaren Kunststoff hergestellt sein.

Die Schale S kann beispielsweise aus Titan hergestellt sein, wohingegen der Einsatz E aus Polyethylen (PE, ISO 5834-2), aus einer Keramik auf Aluminiumoxid-Basis (z.B. eine unter der Marke CERASUL® der Anmelderin erhältliche Keramik, ISO 6474-A), oder aus einer metallischen Kobalt-Chrom-Molybdän Legierung (z.B. eine unter der Marke METASUL® der Anmelderin erhältliche Metall-Legierung, ISO 5832-12) hergestellt sein kann.

## Patentansprüche

1. Instrument (1) zum Einbringen eines Einsatzes (E) eines Implantats, insbesondere einer künstlichen Hüftgelenkpfanne (HP), in die zugehörige Schale (S) dieses Implantats, mit einem Stab (2), der an seinem distalen Ende eine Haltevorrichtung (5) zum Festhalten des Einsatzes (E) aufweist, und mit einem Betätigungsorgan (3,30) zum Freigeben des Einsatzes (E) in die Schale (S), wobei die Haltevorrichtung (5) mindestens drei in axialer Richtung weisende federnde Lamellen (50) umfasst, die so angeordnet sind, dass ihre freien Enden vom Stab (2) weg weisen, und die so ausgebildet sind, dass sie beim Halten des Einsatzes (E) zusammen mit dem Einsatz (E) eine Schnappverbindung bilden, wobei die Lamellen (50) auf ihrer dem Einsatz (E) zugewandten, nach innen weisenden Wand jeweils eine Fläche (500) aufweisen, die gemeinsam eine sich entgegen der Einbringrichtung des Einsatzes (E) verjüngende Konusfläche definieren und zusammen mit einer entsprechenden konischen Fläche (K) auf der Aussenwand des Einsatzes (E) einen festen Sitz bilden.

2. Instrument nach Anspruch 1, **dadurch gekennzeichnet, dass** der Konuswinkel (α) der Konusfläche grösser oder gleich dem Konuswinkel (β) des Konus auf der Aussenwand des Einsatzes (E) ist.

3. Instrument nach einem der Ansprüche 1 oder 2, **dadurch gekennzeichnet, dass** am proximalen Ende der die Konusfläche definierenden Flächen auf der Innenwand der Lamellen (50), also dort, wo der an der Innenwand der Lamellen vorgesehen Konus seinen grössten Durchmesser aufweist, jeweils ein in radialer Richtung nach innen vorstehender Vorsprung (501) vorgesehen ist, der einen Anschlag für den Einsatz (E) bildet.

4. Instrument nach einem der Ansprüche 1 bis 3, **dadurch gekennzeichnet, dass** das distale Ende der die Konusfläche definierenden Fläche der jeweiligen Lamelle (50) mit einer Fase (502) versehen ist.

5. Instrument nach einem der vorstehenden Ansprüche, **dadurch gekennzeichnet, dass** der Stab (2) im wesentlichen hohlzylindrisch ausgebildet ist, und dass das Betätigungsorgan zum Freigeben des Einsatzes eine Schubstange (3) mit einem an ihrem distalen Ende befestigten Stössel (31) aufweist, wobei die Schubstange (3) in dem hohlzylindrischen Stab (2) geführt und in Längsrichtung gegen die Kraft einer Rückstellfeder (4) bewegbar ist, sodass der Stössel (31) bei Betätigung der Schubstange (3) zwischen die Lamellen (50) eintaucht und einen zwischen den Lamellen (50) festgehaltenen Einsatz (E) ausstösst, und anschliessend die Schubstange (3) mitsamt dem Stössel (31) mittels der Rückstellfeder (4) wieder in ihre Ruhestellung bewegt wird.

6. Instrument nach einem der voranstehenden Ansprüche, **dadurch gekennzeichnet, dass** die Haltevorrichtung einen separaten Haltekopf (5) umfasst, der mit dem Stab (2) lösbar verbunden ist und an dem die Lamellen (50) vorgesehen sind.

## Claims

1. An instrument for the introduction of an inlay (E) of an implant, in particular an artificial hip joint socket (HP), into the associated shell (S) of this implant, comprising a bar (2) which has a holder (5) at its distal end for holding the inlay (E), and an actuating member (3, 30) for releasing the inlay (E) into the shell (S), wherein the holder (5) comprises at least three resilient lamellae (50) pointing in the axial direction, which are arranged such that their free ends extend away from the bar (2) and which are shaped such that they form a snap connection together with the inlay (E) on holding the inlay (E), wherein the lamellae (50) each have a surface (500) on their inwardly directed wall confronting the inlay (E), which together define a conical surface tapering against the direction of introduction of the inlay (E) and forming a firm seat together with a corresponding conical surface (K) on an outer wall of the inlay (E).

2. An instrument in accordance with claim 1, **characterised in that** the cone angle (α) of the conical surface is greater than or equal to the cone angle (β) of the cone on the outer wall of the inlay (E).

3. An instrument in accordance with claim 1 or claim 2, **characterised in that** in each case a protrusion (501) projecting inwardly in the radial direction is provided at the proximal end of the surfaces defining the conical surface on the inner wall of the lamellae (50), i.e. where the cone provided at the inner wall of the lamellae has its largest diameter, and forms an abutment for the inlay (E).

4. An instrument in accordance with any of claims 1 to 3, **characterised in that** the distal end of the surface of the respective lamella defining the conical surface is provided with a chamfer (502).

5. An instrument in accordance with any of the preceding claims, **characterised in that** the bar (2) is designed substantially as a hollow cylinder; and **in that** the actuating member for the release of the inlay has a thrust rod (3) with a plunger (31) fastened to its distal end, the thrust rod (3) being guided in the hollow cylindrical bar (2) and being movable in the longitudinal direction against the force of a restoring spring (4) such that the plunger (31) dips between the lamellae (50) when the thrust rod (3) is actuated and ejects an inlay (E) held firmly between the lamellae (50) and the thrust rod (3) together with the plunger (31) is subsequently moved back to its rest position by means of the restoring spring (4).

6. An instrument in accordance with any of the preceding claims, **characterised in that** the holder comprises a separate holder head (5) which is releasably connected to the bar (2) and at which the lamellae (50) are provided.

## Revendications

1. Instrument (1) pour l'introduction d'une pièce d'insertion (E) d'un implant, notamment d'une coque acétabulaire artificielle (HP), dans la coque associée (S) de cet implant, avec une tige (2) qui présente à son extrémité distale un dispositif de retenue (5) pour la retenue de la pièce d'insertion (E), et avec un organe d'actionnement (3, 30) pour libérer l'insert (E) dans la coque (S), où le dispositif de retenue (5) présente au moins trois lamelles élastiques (50) orientées dans la direction axiale qui sont disposées de façon que leurs extrémités libres soient orientées au loin de la tige (2) et qui sont réalisées de façon que lors de la retenue de la pièce d'insertion (E), elles forment ensemble avec la pièce d'insertion (E) une liaison à enclenchement, où les lamelles (50) présentent sur leur paroi orientée vers la pièce d'insertion (E), dirigée vers l'intérieur, respectivement une face (500) qui définissent ensemble une face conique diminuant contre la direction d'introduction de la pièce d'insertion (E) et forment ensemble avec une face conique correspondante (K) sur la paroi extérieure de la pièce d'insertion (E) un ajustement solide.

2. Instrument selon la revendication 1, **caractérisé en ce que** l'angle conique (α) de la face conique est plus grand ou égal à l'angle conique (β) du cône sur la paroi extérieure de la pièce d'insertion (E).

3. Instrument selon l'une des revendications 1 ou 2, **caractérisé en ce qu'**il est prévu à l'extrémité proximale des faces, définissant la face conique, sur la paroi intérieure des lamelles (50), donc à l'endroit où le cône prévu à la paroi intérieure des lamelles présente son plus grand diamètre, respectivement une saillie (501) faisant saillie dans la direction radiale vers l'intérieur qui forme une butée pour la pièce d'insertion (E).

4. Instrument selon l'une des revendications 1 à 3, **caractérisé en ce que** l'extrémité distale de la face définissant la face conique de la lamelle respective (50) est pourvue d'un chanfrein (502).

5. Instrument selon l'une des revendications précédentes, **caractérisé en ce que** la tige (2) est réalisée sensiblement en cylindre creux, et **en ce que** l'organe d'actionnement, pour la libération de la pièce d'insertion, présente une tige de poussée (3) avec un poussoir (31) fixé à son extrémité distale, où la tige de poussée (3) est guidée dans la tige cylindrique creuse (2) et est déplaçable dans la direction longitudinale contre la force d'un ressort de rappel (4) de telle sorte que le poussoir (31), lors de l'actionnement de la tige de poussée (3), plonge entre les lamelles (50) et expulse une pièce d'insertion (E) retenue entre les lamelles (50), et ensuite la tige de poussée (3) est amenée avec le poussoir (31) au moyen du ressort de rappel (4) de nouveau dans sa position de repos.

6. Instrument selon l'une des revendications précédentes, **caractérisé en ce que** le dispositif de retenue comporte une tête de retenue séparée (5) qui est reliée relâchablement à la tige (2) et à laquelle sont prévues les lamelles (50).
